# EUROPÄISCHE PATENTSCHRIFT

(11) **EP 2 811 950 B1**
(45) Veröffentlichungstag und Bekanntmachung des Hinweises auf die Patenterteilung: **16.08.2017**
(21) Anmeldenummer: 12801451.1
(22) Anmeldetag: 22.11.2012
(51) Int. Cl.: A61F 5/37, A61F 5/01

(54) **ORTHESE UND VERFAHREN ZUM ANLEGEN EINER ORTHESE**
ORTHOSIS AND METHOD FOR THE PLACEMENT OF AN ORTHOSIS
ORTHÈSE ET PROCÉDÉ DE POSE D'UNE ORTHÈSE

(30) Priorität: 06.01.2012 DE 102012200165
(43) Veröffentlichungstag der Anmeldung: 17.12.2014
(73) Patentinhaber: OPED AG, 6330 Cham (CH)
(72) Erfinder: ERBE, Florian, 83737 Irschenberg (DE); HOPMANN, Gero, 85521 Riemerling (DE)
(74) Vertreter: advotec.
(86) Internationale Anmeldenummer: PCT/EP2012/004830
(87) Internationale Veröffentlichungsnummer: WO 2013/102472

(56) Entgegenhaltungen:
- WO-A1-2004/054481
- DE-T2- 60 209 459
- DE-T2- 69 616 629
- DE-U1- 8 912 344
- US-A- 4 759 353
- US-A- 5 413 552
- US-B1- 7 037 281

## Beschreibung

Die Erfindung betrifft eine Orthese zur Stützung eines Arms einer Person sowie ein Verfahren zum Anlegen einer Orthese, wobei die Orthese eine Befestigungsvorrichtung zur Befestigung der Orthese an einem Rumpf einer Person, und eine Positioniervorrichtung zur Stützung und Fixierung des Arms, insbesondere Unterarms, Ellenbogens und/oder Oberarms, relativ zu einer dem Arm zugehörigen Schulter einer Person, umfasst, wobei die Positioniervorrichtung von der Befestigungsvorrichtung gehaltert ist, wobei die Befestigungsvorrichtung eine Bauchgurteinrichtung und eine Schultergurteinrichtung umfasst, wobei bei der an einer Person angelegten Orthese die Bauchgurteinrichtung den Rumpf in einer Transversalebene umgibt, und wobei die Schultergurteinrichtung über eine dem Arm abgewandte Schulter der Person verläuft.

Die US 5,413,552 beschreibt eine Armschlinge zur Fixierung eines Unterarms, bei dem die Armschlinge durch einen Schultergurt in der Höhe fixiert werden kann. Außerdem ist an der Armschlinge ein Bauchgurt befestigt, der seinerseits mit einem zweiten Schultergurt in der Höhe fixiert werden kann.

Die US 4,759,353 beschreibt eine Orthese nach dem Oberbegriff des Anspruchs 1, insbesondere eine Armschlinge zur Fixierung eines Unterarms, die mit einem Bauchgurt und einem Schultergurt am Körper des Patienten fixiert wird. Zur Befestigung des Schultergurts am distalen Ende der Armschlinge ist eine Bandschlaufe vorgesehen, die die Armschlinge umgreift.

Die US 7,037,281 B1 beschreibt eine Armschlinge, die mit zwei die beiden Schultern übergreifenden Schultergurten und einem Bauchgurt fixiert wird. Außerdem befinden sich zwischen den beiden Schultergurten kreuzförmig angeordnete Brustgurte.

Derartige Orthesen dienen zur Stabilisierung, Entlastung, Ruhigstellung, Führung oder Korrektur einer Schulter bzw. eines Schultergelenks einer Person. Eine Positioniervorrichtung der Orthese ist dabei immer so ausgebildet, dass ein zu der betreffenden Schulter gehöriger Arm einer Person relativ zur Schulter fixiert oder in seiner Bewegungsfreiheit eingeschränkt wird, um beispielsweise die Schulter zu stabilisieren oder eine Fehlstellung der Schulter zu behandeln. Eine Fixierung eines Arms erfolgt regelmäßig mittels einer Armauflage, die zumindest abschnittsweise entlang eines Unterarms verläuft. Weiter umfassen die bekannten Orthesen eine Befestigungsvorrichtung, die zur Verbindung der Positioniervorrichtung mit einem Rumpf der betreffenden Person dient. Die Befestigungsvorrichtung ist regelmäßig aus Riemen oder Gurten gebildet, welche an die Körperproportionen der betreffenden Person angepasst werden können, und die die Positioniervorrichtung bzw. die Armauflage mit dem daran fixierten Arm der Person an deren Rumpf befestigen bzw. relativ dazu in der gewünschten Position fixieren.

Da eine mit einer derartigen Orthese zu behandelnde Person die Orthese oftmals über einen vergleichsweise langen Zeitraum von mehreren Wochen tragen muss, ist es erforderlich, dass die Orthese von der betreffenden Person selbst leicht gehandhabt werden kann und die Person so wenig wie möglich in ihrer Lebensführung eingeschränkt wird. Insbesondere die am Körper der Person verlaufenden Gurte oder Riemen werden häufig als störend empfunden. Wenn beispielsweise die Orthese auch nachts im Bett getragen werden muss, können die Riemen oder Gurte eine Bewegungsfreiheit wesentlich einschränken und störende Druckstellen am Körper der Person bewirken. Auch kann die Orthese so ausgebildet sein, dass die betreffende Person für beispielsweise einen Kleidungswechsel die Orthese nur mit Hilfe einer weiteren Person ablegen und anlegen kann. Dies ist insbesondere dadurch begründet, dass der zur behandelten Schulter gehörige Arm nicht für diesen Vorgang benutzt werden, und somit ein Anlegen der Orthese nur einhändig erfolgen kann. Gleiches betrifft eventuelle Verstelleinrichtungen der Orthese zur Lageverstellung des zu fixierenden Arms.

Der vorliegenden Erfindung liegt daher die Aufgabe zu Grunde, eine Orthese zur Stützung eines Arms einer Person und ein Verfahren zum Anlegen einer derartigen Orthese vorzuschlagen, die bzw. mit dem eine für eine Person komfortablere Nutzung ermöglicht wird.

Diese Aufgabe wird durch eine Vorrichtung mit den Merkmalen des Anspruchs 1 und ein Verfahren mit den Merkmalen des Anspruchs 12 gelöst.

Die Erfindung betrifft gattungsgemäß eine Orthese zur Stützung eines Arms einer Person, umfassend eine Befestigungsvorrichtung zur Befestigung der Orthese an einem Rumpf der Person, und eine Positioniervorrichtung zur Stützung und Fixierung des Arms, insbesondere Unterarms, Ellenbogens und/oder Oberarms, relativ zur einer dem Arm zugehörigen Schulter einer Person, wobei die Positioniervorrichtung von der Befestigungsvorrichtung gehaltert ist, wobei die Befestigungsvorrichtung eine Bauchgurteinrichtung und eine Schultergurteinrichtung umfasst, wobei bei der an einer Person angelegten Orthese die Bauchgurteinrichtung den Rumpf in einer Transversalebene umgibt, und wobei die Schultergurteinrichtung über eine dem Arm abgewandte Schulter der Person verläuft, wobei die Schultergurteinrichtung einen Schultergurt und einen damit verbundenen Brustgurt umfasst, die an einem distalen Ende und einem proximalen Ende der Positioniervorrichtung, sowie an einem ventralen Abschnitt der Bauchgurteinrichtung angelenkt sind.

Folglich umfasst die Befestigungsvorrichtung eine Bauchgurteinrichtung, einen Schultergurt und einen Brustgurt. Die Bauchgurteinrichtung ist so ausgebildet, dass die an einer Person angelegte Orthese den Rumpf einer Person im Wesentlichen im Bereich des Bauches oder der Hüften umfänglich umgibt. Die Bauchgurteinrichtung ist weiter so mit der Positioniervorrichtung verbunden, dass diese am Rumpf angeordnet ist. Der Schultergurt ist an einem proximalen Ende der Positioniervorrichtung angelenkt und verläuft quer über den Rücken und die dem fixierten Arm abgewandte Schulter der Person bis zumindest auf eine Frontseite des Rumpfes. Im Bereich der Brust der Person wird die Schultergurteinrichtung durch zwei Gurtabschnitte fortgeführt, die durch den Schultergurt und den Brustgurt oder den Brustgurt alleine ausgebildet sein können. Ein Gurtabschnitt ist an einem distalen Ende der Positioniervorrichtung und der weitere Gurtabschnitt an einem ventralen Abschnitt der Bauchgurteinrichtung angelenkt. Durch diese Anordnung der betreffenden Gurte wird insbesondere der Vorteil erzielt, dass im Bereich des Rückens der Schultergurt ohne eine Knotenverbindung oder Schnalle verläuft. Auch ist kein weiterer Gurt bzw. Riemen unterhalb des der betreffenden Schulter gegenüberliegenden Arms erforderlich. Weiter ist durch die Verbindung der Schultergurteinrichtung mit dem distalen Ende der Positioniervorrichtung ein Spannen der Schultergurteinrichtung und damit ein Heranziehen der Positioniervorrichtung bzw. der Armauflage an den Rumpf möglich. Durch die Verbindung der Schultergurteinrichtung mit dem ventralen Abschnitt der Bauchgurteinrichtung wird weiter ein Verlauf des Schultergurtes und des Brustgurtes im Brustbereich vorteilhaft beeinflusst. Dies ist insbesondere dann von Bedeutung, wenn die Orthese von weiblichen Personen getragen werden soll, da von diesen über die Brust verlaufende Gurte häufig als störend empfunden werden. Weiter ist es möglich aufgrund der vorbeschriebenen Ausbildung der Schultergurteinrichtung, die Orthese besonders einfach einhändig anzulegen bzw. abzulegen, ohne dass eine weitere Person notwendig wäre.

Erfindungsgemäß verläuft der Brustgurt zwischen dem distalen Ende der Positioniervorrichtung und dem ventralen Abschnitt der Bauchgurteinrichtung. So kann dann der Schultergurt im Bereich der Brust einer Person an den Brustgurt angelenkt sein. Beispielsweise kann dann ein Verbindungsabschnitt von Schultergurt und Brustgurt durch entsprechende Längenvariationen der Gurtabschnitte so im Brustbereich platziert werden, dass die Schultergurteinrichtung nicht störend wirkt.

Weiter ist vorgesehen, dass der Schultergurt zwischen dem proximalen Ende der Positioniervorrichtung und dem Brustgurt verlaufen. So wird es möglich, eine Gurtlänge der Schultergurteinrichtung zwischen dem distalen Ende und dem proximalen Ende der Positioniervorrichtung durch eine längenveränderliche Einstellung des Schultergurtes oder des Brustgurtes zu beeinflussen.

Die Positioniervorrichtung kann erfindungsgemäß eine Stützeinrichtung aufweisen, die an einem lateralen Abschnitt der Bauchgurteinrichtung befestigt ist, und die Positioniervorrichtung kann weiter eine Armauflage aufweisen, wobei an der Armauflage ein Arm einer Person fixierbar ist, wobei die Stützeinrichtung mit der Armauflage mittels einer Verbindungseinrichtung verbunden sein kann. Wenn die Stützeinrichtung an einem lateralen Abschnitt der Bauchgurteinrichtung befestigt ist, befindet sie sich im Wesentlichen direkt unterhalb eines gebeugten Unterarmes. Die Stützeinrichtung kann beispielsweise plattenförmig ausgebildet sein, und im Wesentlichen in einer Horizontalebene an der Bauchgurteinrichtung befestigt sein. Eine Verbindung zwischen der Stützeinrichtung und der Bauchgurteinrichtung kann über an der Bauchgurteinrichtung angeformte oder angeflanschte Verbindungselementen erfolgen. Die Armauflage der Positioniervorrichtung kann so ausgebildet sein, dass ein Arm einer Person an der Armauflage besonders leicht fixierbar ist. Beispielsweise kann die Armauflage in der Art einer Schiene oder als ein trogförmiges Element, welches den betreffenden Arm zumindest bereichsweise aufnimmt, ausgebildet sein. So kann die Armauflage auch eine Aufnahme eines Ellenbogens umfassen, und damit eine definierte Positionierung des Arms in der Armauflage gewährleisten. Vorzugsweise kann die Armauflage auch Riemen oder Gurte umfassen, mittels derer ein Unter- und/oder Oberarm an der Armauflage leicht fixiert werden kann. Weiter ist es auch möglich, dass die Armauflage über eine Polsterung verfügt, um eventuelle Druckstellen zu vermeiden. Die Verbindungseinrichtung kann dann die Armauflage mit der Stützeinrichtung verbinden, wobei die Verbindungseinrichtung durch die Armauflage und die Stützeinrichtung selbst ausgebildet sein kann. Die Verbindungseinrichtung kann jedoch auch ein eigenen Bauteil oder eine Baugruppe sein, die zwischen der Armauflage und der Stützeinrichtung angeordnet ist. Besonders vorteilhaft ist es, wenn der Schultergurt an dem verbundenen Brustgurt entlang des Brustgurtes verschiebbar anordenbar ist. Die Orthese kann dann besonders einfach einhändig angelegt werden, wenn eine Person zunächst den Schultergurt über die dem fixierten Arm abgewandte Schulter legt. Dies kann durch ein Hineinschlüpfen in den Schultergurt, wie bei einer Jacke, erfolgen. Da dann der Schultergurt an dem Brustgurt verschiebbar anordenbar ist, ist die Schultergurteinrichtung noch nicht gespannt, wodurch das Anlegen des Schultergurts vereinfacht wird. Nach dem Anlegen des Schultergurts kann der Brustgurt an dem distalen Ende der Positioniervorrichtung befestigt werden. Durch diese Befestigung, welche einhändig ausgeführt werden kann, erfolgt dann ein Spannen des Schultergurts. Der Brustgurt kann demnach längeneinstellbar sein.

Weiter ist es möglich, einen Verlauf des Schultergurts bzw. des Brustgurts im Brustbereich vorteilhaft anzupassen, wenn der Brustgurt an einem Bauchgurt der verbundenen Bauchgurteinrichtung entlang des Bauchgurtes in dem ventralen Abschnitt verschiebbar angeordnet ist. In diesem Fall kann der Brustgurt bzw. eine Position, an der der Brustgurt mit dem Bauchgurt verbunden ist, entlang des Bauchgurtes verschoben werden. Die Orthese wird damit besonders universell einsetzbar, da der Brustgurt unabhängig von einem Rumpfumfang bzw. Bauchumfang einer Person und damit unabhängig von einer Länge des Bauchgurtes immer in einem Mittenbereich des ventralen Abschnittes bzw. in einer Sagittalebene des Rumpfes durch Verschieben angeordnet werden kann.

Vorzugsweise kann die Verbindungseinrichtung so ausgebildet sein, dass die Armauflage relativ zur Stützeinrichtung lösbar fixierbar ist. Da die Stützeinrichtung durch ihre Fixierung an einem Rumpf mittels der Bauchgurteinrichtung relativ zu dem Rumpf weitestgehend in ihrer Position festgelegt ist, ist es dann möglich, die Armauflage, und damit den betreffenden Arm, relativ zum Rumpf in der gewünschten Position auszurichten und zu fixieren. Vorzugsweise können hier Befestigungsmittel vorgesehen sein, die ein wiederholtes Lösen und Befestigen ermöglichen und die einfach mit einer Hand bedienbar sind.

An der Armauflage kann eine Handauflage angeordnet sein, die relativ zur Armauflage in einer Längsrichtung der Armauflage verschiebbar ist. Insbesondere bei einer gebeugten Position eines Arms kann so eine zum Arm gehörige Hand durch die Handauflage abgestützt werden. Durch eine Verschiebbarkeit der Handauflage kann weiter eine Anpassung der Handauflage an eine individuelle Armlänge erfolgen. Die Handauflage kann beispielsweise in einer Längsführung der Armauflage befestigt sein, und mittels mit einer Hand bedienbaren Rastelementen in Längsrichtung der Armauflage gelöst, verschoben und fixiert werden.

Die Verbindungseinrichtung kann so ausgebildet sein, dass die Armauflage relativ zur Stützeinrichtung bewegbar ist. Hierdurch kann der Vorteil erzielt werden, dass trotz einer Befestigung der Armauflage an der Stützeinrichtung eine Armbewegung in definierten Grenzen möglich ist. So können beispielsweise durch eine begrenzte Armbewegung bestimmte Muskelgruppen trainiert werden, ohne dass die zu behandelnde Schulter durch die Bewegung geschädigt wird. Auch kann ein Arm dann einerseits vollständig fixiert werden und wahlweise in einer durch die Verbindungseinrichtung vorgegebenen Bewegungsrichtung zu Trainingszwecken bewegt werden.

So kann von der Verbindungseinrichtung und der Stützeinrichtung eine Linearführung ausgebildet sein, wobei die Linearführung so angeordnet sein kann, dass eine Abduktion bzw. Adduktion der Armauflage relativ zu der Stützeinrichtung bzw. zu einer Sagittalebene des Rumpfs möglich ist. Folglich kann ein Oberarm relativ zum Rumpf gebeugt oder gestreckt werden, wobei dann die Armauflage eine im Wesentlichen lineare Bewegung relativ zur Stützeinrichtung ausführt. Eine Bewegung des Arms relativ zu einer Frontalebene des Rumpfs ist dann aufgrund einer durch die Linearführung bewirkte Bewegungseinschränkung des Arms nicht möglich.

Um die Linearführung gegebenenfalls verlängerbar auszubilden, kann an die Stützeinrichtung ein Führungsabschnitt adaptierbar sein, mittels dem die Linearführung verlängert werden kann. Wenn die Stützeinrichtung beispielsweise in der Art einer Platte ausgebildet ist, kann an der Stützeinrichtung einfach eine weitere Platte befestigt werden, die die Stützeinrichtung und damit die Linearführung verlängert. Die Linearführung kann beispielsweise als eine Ausnehmung in der Stützeinrichtung oder als eine schlitzförmige Durchgangsöffnung ausgebildet sein. Weiter können Rastvorsprünge an der Linearführung ausgebildet sein, mit denen die Verbindungseinrichtung in den gewünschten Positionen verrastet und damit fixiert werden kann.

Weiter kann die Verbindungseinrichtung eine Rotationsführung ausbilden, mit der eine Rotation der Armauflage in einer Transversalebene des Rumpfs möglich wird. Folglich ist es dann nicht erforderlich, die Armauflage parallel zu bzw. in Richtung einer Sagittalebene des Rumpfs auszurichten, sondern in verschiedenen Winkelpositionen relativ zur Sagittalebene zu fixieren und/oder in vorgegebenen Grenzen zu bewegen. Wie bei der Linearführung wird so mit der Rotationsführung ein Training von Muskelgruppen und eine bedarfsgerechte Positionierung und Fixierung des betreffenden Arms möglich.

Vorzugsweise kann die Rotationsführung eine Rotation in einem Bereich von +30° bis -30° relativ zu der Stützeinrichtung bzw. zu einer Sagittalebene des Rumpfs ermöglichen. Weiter können an der Rotationsführung Rastelemente ausgebildet sein, die ein Verrasten und damit Fixieren der Armauflage in bestimmten Winkelschritten, von beispielsweise 5°, ermöglichen.

Um einen sicheren Halt der Armauflage relativ zum Rumpf bei gleichzeitig einfacher Handhabung zu gewährleisten, können der Schultergurt und der Brustgurt jeweils mit der Armauflage lösbar verbindbar sein. Folglich kann der Schultergurt dann an dem proximalen Ende der Armauflage und der Brustgurt an dem distalen Ende der Armauflage befestigt werden. Eine lösbare Befestigung kann einfach durch eine Haken-, eine Schnallen- oder eine Steckverbindung realisiert werden. So kann dann der Brustgurt bei einem Anlegen der Orthese auch einfach mit einer Hand mit der Armauflage verbunden werden. Weiter wird dann ein Gewicht des Arms über die Schultergurteinrichtung unmittelbar über der dem Arm abgewandte Schulter abgestützt.

Insofern kann es auch vorteilhaft sein, wenn die Verbindungseinrichtung eine Kippführung ausbildet, derart, dass ein Kippen der Armauflage relativ zu der Stützeinrichtung bzw. zu einer Transversalebene des Rumpfs möglich ist. Ein Unterarm kann dann relativ zu der Transversalebene in einem Winkel geneigt bzw. gestreckt oder angewinkelt werden. Die Kippführung ermöglicht dann eine Bewegung des Unterarms in vorgegebenen Grenzen oder eine verbesserte Anpassung einer gewünschten Position des Unterarms.

Ein Kippen der Armauflage kann mittels einer Verstellung des Schultergurts oder des Brustgurts ermöglicht werden. So kann die Position des Unterarms relativ zur Transversalebene leicht eindeutig festgelegt und an individuelle Körperabmessungen angepasst werden.

Bei dem erfindungsgemäßen Verfahren zum Anlegen einer Orthese zur Stützung eines Arms einer Person umfasst die Orthese eine Befestigungsvorrichtung zur Befestigung der Orthese an einem Rumpf der Person, und eine Positioniervorrichtung zur Stützung und Fixierung des Arms, wobei die Positioniervorrichtung von der Befestigungsvorrichtung gehaltert wird, und wobei die Befestigungsvorrichtung eine Bauchgurteinrichtung und eine Schultergurteinrichtung umfasst, wobei der Arm an der Positioniervorrichtung fixiert wird, wobei die Bauchgurteinrichtung am Rumpf, diesen in einer Transversalebene umgebend, fixiert wird, wobei ein Schultergurt der Schultergurteinrichtung, der mit einem proximalen Ende der Positioniervorrichtung verbunden ist, über eine dem Arm abgewandte Schulter der Person gezogen wird, und wobei ein mit dem Schultergurt verbundener Brustgurt der Schultergurteinrichtung, der an einem ventralen Abschnitt der Bauchgurteinrichtung angelenkt ist, mit einem distalen Ende der Positioniervorrichtung verbunden wird.

Insbesondere durch die Ausbildung des Schultergurtes und Brustgurtes sowie deren Befestigung an der Bauchgurteinrichtung und an der Positioniervorrichtung wird es möglich, die Orthese besonders leicht einhändig anzulegen und einzustellen, ohne dass dazu der mit der Orthese abgestützte Arm benutzt werden müsste. Insbesondere kann dies dadurch erreicht werden, dass der Brustgurt maximal verlängert werden kann, und so eine größtmögliche Schlaufe bildet, durch die mit dem Oberkörper hindurch geschlüpft werden kann.

Auch kann der Arm durch eine Verstellung des Brustgurtes in seiner Länge ausbalanciert werden. D.h. der Brustgurt kann so in seiner Länge eingestellt bzw. verstellt werden, dass der Arm bzw. die Armauflage so angeordnet bzw. ausbalanciert ist, dass ein Schwerpunkt des Arms bzw. eines Schulter-Arm-Systems so positioniert ist, dass der Arm sicher auf der Armauflage aufliegt ohne zu verrutschen. Insbesondere kann die Armauflage waagerecht angeordnet sein.

Weiter wird auf die Vorteilsbeschreibung zu der erfindungsgemäßen Orthese verwiesen. Vorteilhafte Ausführungsformen des Verfahrens ergeben sich aus den auf den Vorrichtungsanspruch 1 rückbezogenen Unteransprüchen.

Nachfolgend wird eine bevorzugte Ausführungsform der Erfindung unter Bezugnahme auf die beigefügte Zeichnung näher erläutert.

Es zeigen:
- **Fig. 1**: eine perspektivische Ansicht einer Orthese;
- **Fig. 2**: die Orthese in einer Seitenansicht von links bzw. einer Ansicht einer Sagittalebene;
- **Fig. 3**: die Orthese in einer Vorderansicht bzw. einer Ansicht einer Frontalebene;
- **Fig. 4**: die Orthese in einer Rückansicht bzw. einer Ansicht der Frontalebene;
- **Fig. 5**: die Orthese in einer Draufsicht bzw. einer Ansicht einer Transversalebene;
- **Fig. 6**: die Orthese in einer Unteransicht bzw. in einer Ansicht der Transversalebene;
- **Fig. 7**: eine Stützeinrichtung der Orthese in einer perspektivischen Ansicht;
- **Fig. 8**: die Stützeinrichtung der Orthese in einer Draufsicht.

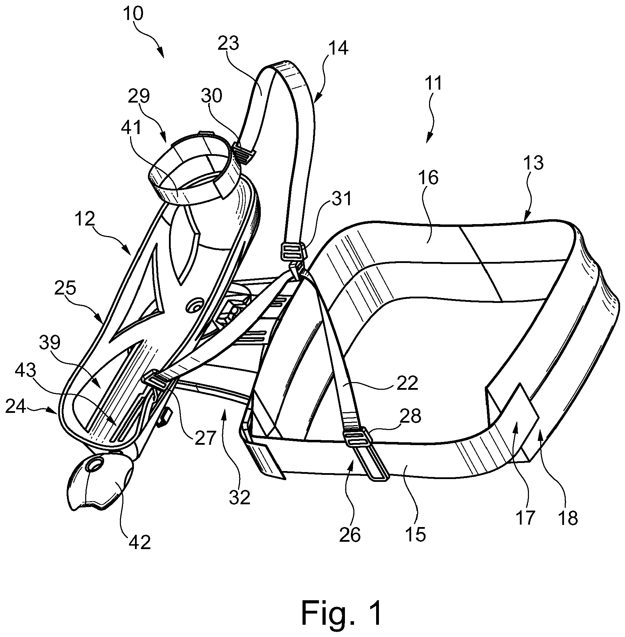

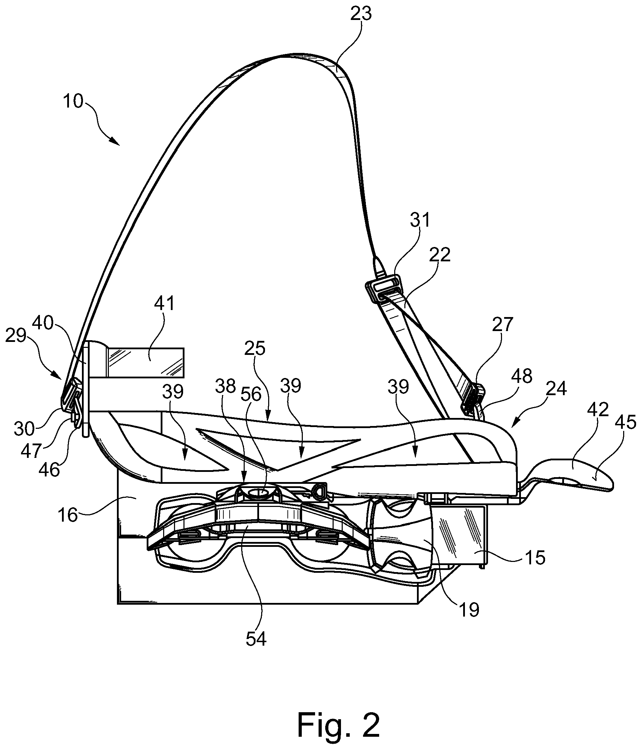

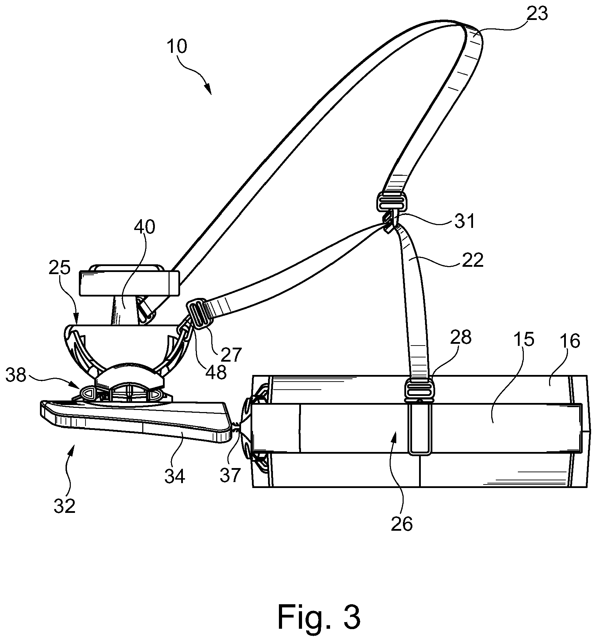

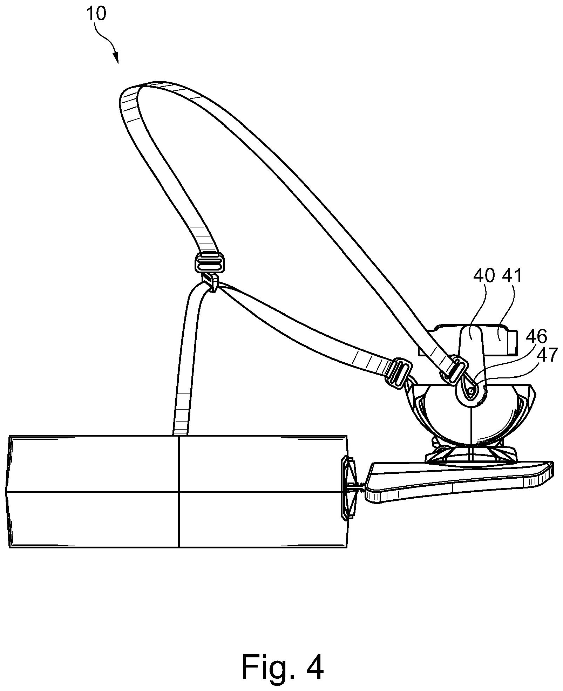

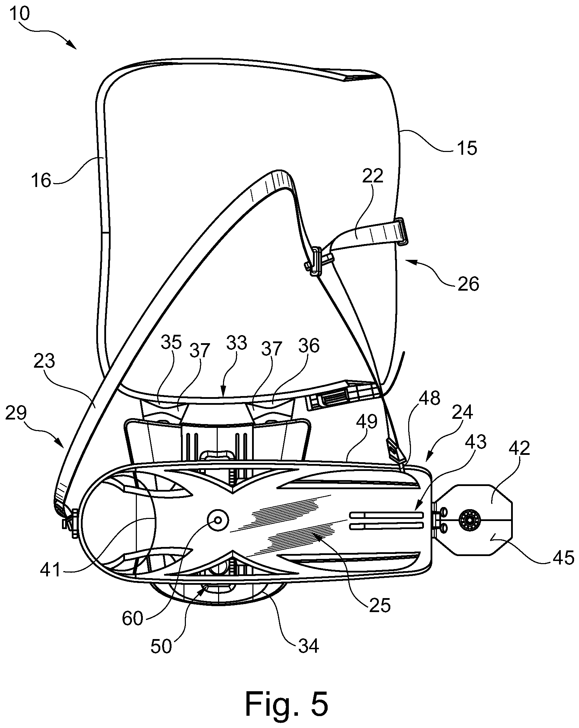

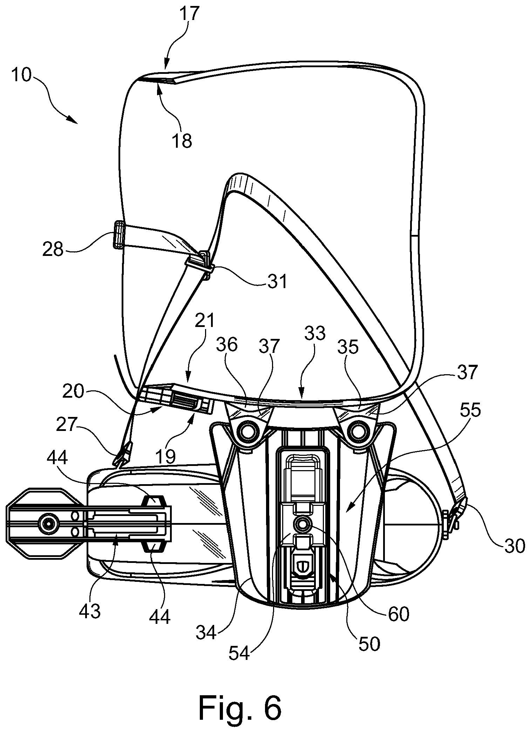

Eine Zusammenschau der **Fig. 1** bis **6** zeigt eine Ausführungsform einer Orthese 10 in verschiedenen Ansichten. Die Orthese 10 ist so dargestellt, wie sie an einem hier nicht gezeigten Rumpf einer Person angeordnet bzw. an der Person angelegt ist. Die Orthese 10 besteht im Wesentlichen aus einer Befestigungsvorrichtung 11 und einer Positioniervorrichtung 12, wobei die Befestigungsvorrichtung 11 zur Befestigung der Orthese 10 an dem Rumpf der Person, und die Positioniervorrichtung 12 zur Stützung und Fixierung eines hier nicht dargestellten Arms der Person relativ zu einer dem Arm zugehörigen Schulter dient. Die Befestigungsvorrichtung 11 haltert dabei die Positioniervorrichtung 12 am Rumpf der Person. Die Befestigungsvorrichtung 11 umfasst weiter eine Bauchgurteinrichtung 13 und eine Schultergurteinrichtung 14. Die Bauchgurteinrichtung 13 ist aus einem flexiblen Bauchgurt 15, bestehend aus einem Gewebematerial, und aus einem überwiegend starren Beckengurt 16 gebildet. Der Beckengurt 16 besteht aus einem teilflexiblen Kunststoffmaterial, wodurch er sich an eine Körperform der Person anpassen und gleichzeitig eine vergleichsweise starre Halterung für die Positioniervorrichtung 12 ausbilden kann. Der Bauchgurt 15 ist mit einem Ende 17 an einem Ende 18 des Beckengurts 16 unlösbar befestigt, und mit einer Hakenschnalle 19 an einem Ende 20 des Bauchgurtes bzw. einem Ende 21 des Beckengurtes 16 lösbar mit diesem verbunden. Weiter ist es möglich, mittels der Hakenschnalle 19 eine Länge des Bauchgurtes 15 zu variieren und diesen damit an unterschiedliche Körpermaße anzupassen.

Die Schultergurteinrichtung 14 ist im Wesentlichen aus einem Brustgurt 22 und einem Schultergurt 23 gebildet. Der Brustgurt 22 ist an einem distalen Ende 24 einer Armauflage 25 der Positioniervorrichtung 12 befestigt und verläuft bis zu einem ventralen Abschnitt 26 des Bauchgurtes 15. Der Brustgurt 22 ist dabei mittels einer Schnalle 27 am distalen Ende 24 der Armauflage 25 befestigbar und längeneinstellbar. Somit ist durch die Längeneinstellbarkeit auch ein Ausbalancieren des Armes bzw. der Armauflage 25 möglich. Weiter ist der Brustgurt 22 mittels einer Schnalle 28 mit dem Bauchgurt 15 verbunden, wobei die Schnalle 28 entlang des Bauchgurts 15 verschiebbar anordenbar ist. Die Schnalle 28 kann somit immer so angeordnet werden, dass diese sich im Wesentlichen in einer Sagittalebene des Rumpfs befindet. Der Schultergurt 23 ist an einem proximalen Ende 29 der Armauflage 25 mittels einer Schnalle 30 befestigt und verläuft über eine hier nicht dargestellte, eine dem Arm abgewandte Schulter der Person bis hin zum Brustgurt 22 und ist mit diesem mittels einer Schnalle 31 verbunden. Der Schultergurt 23 ist ebenfalls mittels der Schnallen 30 und 31 längenveränderlich. Weiter kann der Schultergurt 23 mittels der Schnalle 31 entlang des Brustgurtes 22 verschoben werden. Darüber hinaus sind die Schnallen 27, 28 und 30, 31 als drehbare Schnallen ausgebildet, dass heißt diese können sich in sich verdrehen, so dass ein unerwünschtes Verdrehen des Brustgurtes 22 und des Schultergurts 23 reversibel ist.

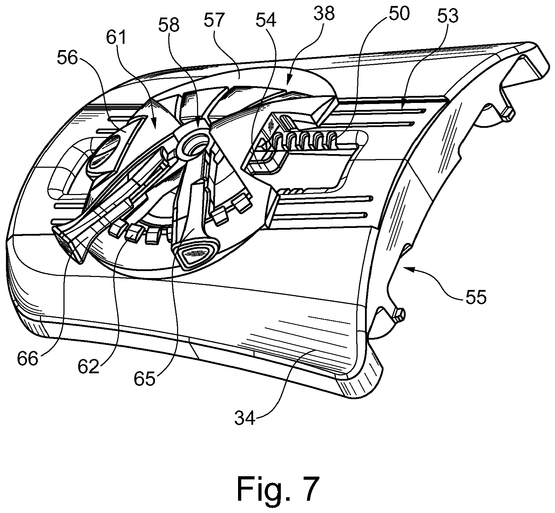

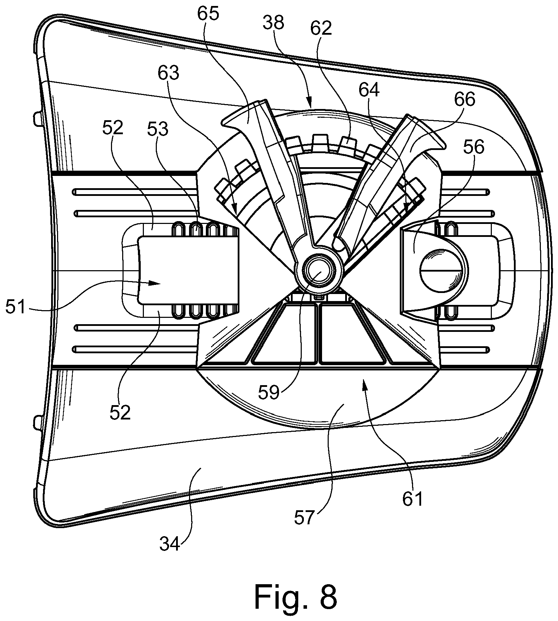

Die in den **Fig. 7** und **8** näher dargestellte Positioniervorrichtung 12 weist eine Stützeinrichtung 32 auf, die an einem lateralen Abschnitt 33 der Bauchgurteinrichtung 13 bzw. am Beckengurt 16 befestigt ist. Die Stützeinrichtung 32 ist im Wesentlichen aus einer Platte 34 mit Halteflanschen 35 und 36 gebildet. Die Halteflansche 35 und 36 sind unmittelbar an dem Beckengurt 16 befestigt und verbinden diesen mit der Platte 34 über jeweils ein Scharnier 37. Somit kann die Platte 34 relativ zur Sagittalebene des Rumpfs nach oben oder nach unten gekippt werden. Weiter umfasst die Positioniervorrichtung 12 eine Verbindungseinrichtung 38, mittels der die Armauflage 25 an der Platte 34 bzw. der Stützeinrichtung 32 befestigt werden kann. Die Armauflage 25 ist in dem hier gezeigten Beispiel trogförmig ausgebildet und damit weitestgehend einer Form eines Unterarms angepasst. Das proximale Ende 29 der Armauflage 25 ist zur positionierenden Aufnahme eines Ellenbogens geschlossen ausgebildet. Weiter weist die Armauflage 25 eine Mehrzahl von unregelmäßigen Durchbrechungen 39 auf, durch die die Armauflage 25 abschnittsweise flexibel ist. Weiter ist vorgesehen, in die Armauflage 25 eine hier nicht dargestellte Polsterung einzulegen, die eine bequeme Auflage des betreffenden Arms ermöglicht. Die Armauflage 25 weist weiter an dem proximalen Ende 29 einen Schalenfortsatz 40 mit einem Oberarmgurt 41 auf. Mittels des Oberarmgurts 41 kann der Oberarm bzw. der Ellenbogen in einer Längsrichtung der Armauflage 25 fixiert werden. Auch kann an dem distalen Ende 24 der Armauflage 25 ein weiterer, hier nicht dargestellter Gurt zur Fixierung des Unterarms an der Armauflage 25 vorgesehen sein. An dem distalen Ende 24 der Armauflage 25 ist weiter eine Handauflage 42 angeordnet, die relativ zur Armauflage 25 in deren Längsrichtung verschiebbar ist. Die Handauflage 42 ist in einer Linearführung 43 der Armauflage 25 längsverschieblich aufgenommen, wobei mittels Rastnasen 44 der Handauflage 42 ein abschnittsweises Verrasten und damit Fixieren der Handauflage 42 in der Linearführung 43 möglich ist. Für eine Verstellung der Handauflage 42 ist es daher ausreichend, die Rastnasen 44 zusammenzudrücken und gleichzeitig die Handauflage 42 in der Linearführung 43 in die gewünschte Position zu verschieben. Die Handauflage 42 ist als ein einstückiges Kunststoffelement ausgebildet und weist eine palmare Auflagefläche 45 für eine Handfläche auf.

Die Schnalle 30 des Schultergurtes 23 ist mittels einer Hakenöse 46 mit einem am proximalen Ende 29 der Armauflage 25 angeformten Nippel 47 verbunden. Die Schnalle 27 des Brustgurtes 22 ist mit einer Hakenöse 48 an dem distalen Ende 24 mit einem Wandungsabschnitt 49 der Armauflage 25 verbunden. Bei gelöster Hakenöse 48 kann der Brustgurt 22 so durch die Schnalle 31 gleiten, dass eine Länge der Schultergurteinrichtung 14 vom proximalen Ende 29 bis zum ventralen Abschnitt 26 maximal verlängert wird, wodurch die Orthese 10 besonders einfach ab- oder angelegt werden kann. Durch ein Verbinden der Hakenöse 48 mit dem Wandungsabschnitt 49 kann die Schultergurteinrichtung 14 wieder einfach gespannt werden.

Die Verbindungseinrichtung 38 ist zwischenliegend der Armauflage 25 und der Platte 34 angeordnet. Die Verbindungseinrichtung 38 bildet zusammen mit der Platte 34 eine Linearführung 50 aus. So ist in der Platte 34 eine Längsausnehmung 51 ausgebildet, die an ihren seitlichen Rändern 52 eine Reihe von Rastausnehmungen 53 aufweist. Die Verbindungseinrichtung 38 ist durch die Längsausnehmung 51 hindurch geführt und hintergreift die Ränder 52 mittels eines Klemmstücks 54 auf einer Unterseite 55 der Platte 34. Die Verbindungseinrichtung 38 kann somit in Längsrichtung der Längsausnehmung 51 verschoben werden und mittels eines Raststücks 56 in der jeweiligen Position fixiert werden. Das Raststück 56 ist an einem Verbindungseinrichtungskörper 57 der Verbindungseinrichtung 38 angelenkt und kann mit den Rastausnehmungen 53 durch Handbetätigung verrasten. Das hier im verrasteten Zustand gezeigte Raststück 56 kann demnach aus den Rastausnehmungen 53 gelöst werden, so dass der Verbindungseinrichtungskörper 57 entlang der Längsausnehmung 51 vollkommen frei bewegbar ist. So kann einerseits eine bedürfnisgerechte Einstellung der Armauflage 25 und deren Fixierung mittels Einhandbedienung erfolgen, und andererseits auch eine Relativbewegung der Armauflage 25 zur Stützeinrichtung 32 in Richtung der Linearführung 50 zum Training bestimmter Muskelgruppen durchgeführt werden.

Die Verbindungseinrichtung 38 weist weiter eine Rotationsführung 58 auf, welche im Wesentlichen aus einer Nabe 59 im Verbindungseinrichtungskörper 57 und einer Achse 60 der Armauflage 25 gebildet ist. Auf einer Oberseite 61 des Verbindungseinrichtungskörpers 57 sind ausgehend von der Nabe 59 Rastnasen 62 in einem Abstand von 5 Winkelgrad ausgebildet. Die Rastnasen 62 werden durch Anschlagflanken 63 und 64 begrenzt. Eine Bewegung der Armauflage 25 ist damit in einem Bereich von 60 Winkelgrad zwischen den Anschlagflanken 63 und 64 möglich. Die Bewegung bzw. Rotation der Armauflage 25 relativ zu der Sagittalebene des Rumpfs kann folglich in einem Bereich von +30 bis -30 Winkelgrad erfolgen. Auch kann die Bewegung der Armauflage 25 auf einen Bereich begrenzt bzw. vollkommen unterbunden werden. Dies erfolgt mittels zweier Anschlagelemente 65 und 66. Die Anschlagelemente 65 und 66 sind jeweils an der Nabe 59 drehbar gehaltert und können im Bereich der Rastnasen 62 um die Nabe 59 verschwenkt werden. Weiter können die Anschlagelemente 65 und 66 durch Verrasten mit den Rastnasen 62 in ihrer jeweiligen Position fixiert werden. Die Anschlagelemente 65 und 66 begrenzen dann eine Bewegung der Armauflage 25. Liegen die Anschlagelemente 65 und 66 aneinander an, ist die Armauflage 25 fixiert und nicht mehr drehbar. Die Anschlagelemente 65 und 66 können mittels Einhandbedienung verstellt werden.

## Patentansprüche

1. Orthese (10) zur Stützung eines Arms einer Person, umfassend eine Befestigungsvorrichtung (11) zur Befestigung der Orthese an einem Rumpf der Person, und eine Positioniervorrichtung (12) zur Stützung und Fixierung des Arms, insbesondere Unterarms, Ellenbogens und/oder Oberarms, relativ zu einer dem Arm zugehörigen Schulter einer Person, wobei die Positioniervorrichtung von der Befestigungsvorrichtung gehaltert ist, wobei die Befestigungsvorrichtung eine Bauchgurteinrichtung (13) und eine Schultergurteinrichtung (14) umfasst, wobei bei der an einer Person angelegten Orthese die Bauchgurteinrichtung den Rumpf in einer Transversalebene umgibt, und wobei die Schultergurteinrichtung über eine dem Arm abgewandte Schulter der Person verläuft, wobei
die Schultergurteinrichtung einen Schultergurt (23) und einen damit verbundenen Brustgurt (22) umfasst, die an einem distalen Ende (24) und einem proximalen Ende (29) der Positioniervorrichtung, sowie an einem ventralen Abschnitt (26) der Bauchgurteinrichtung angelenkt sind, wobei der Schultergurt (23) zwischen dem proximalen Ende (29) der Positioniervorrichtung (12) und dem Brustgurt (22) verläuft,
**dadurch gekennzeichnet,**
**dass** der Brustgurt (22) zwischen dem distalen Ende (24) der Positioniervorrichtung (12) und dem ventralen Abschnitt (26) der Bauchgurteinrichtung (13) verläuft, und wobei
die Positioniervorrichtung (12) eine Stützeinrichtung (32) aufweist, die an einem lateralen Abschnitt (33) der Bauchgurteinrichtung (13) befestigt ist, und dass die Positioniervorrichtung eine Armauflage (25) aufweist, wobei an der Armauflage ein Arm einer Person fixierbar ist, und wobei die Stützeinrichtung mit der Armauflage mittels einer Verbindungseinrichtung (38) verbunden ist.

2. Orthese nach einem der vorangehenden Ansprüche,
**dadurch gekennzeichnet,**
**dass** der Schultergurt (23) an dem verbundenen Brustgurt (22) entlang des Brustgurtes verschiebbar anordbar ist.

3. Orthese nach einem der vorangehenden Ansprüche,
**dadurch gekennzeichnet,**
**dass** der Brustgurt (22) längeneinstellbar ist.

4. Orthese nach einem der vorangehenden Ansprüche,
**dadurch gekennzeichnet,**
**dass** der Brustgurt (22) an einem Bauchgurt (15) der verbundenen Bauchgurteinrichtung (13) entlang des Bauchgurtes in dem ventralen Abschnitt (26) verschiebbar anordbar ist.

5. Orthese nach einem der vorangehenden Ansprüche,
**dadurch gekennzeichnet,**
**dass** die Verbindungseinrichtung (38) so ausgebildet ist, dass die Armauflage (25) relativ zur Stützeinrichtung (32) lösbar fixierbar ist.

6. Orthese nach einem der vorangehenden Ansprüche,
**dadurch gekennzeichnet,**
**dass** an der Armauflage (25) eine Handauflage (42) angeordnet ist, die relativ zur Armauflage in einer Längsrichtung der Armauflage verschiebbar ist.

7. Orthese nach einem der vorangehenden Ansprüche,
**dadurch gekennzeichnet,**
**dass** die Verbindungseinrichtung (38) so ausgebildet ist, dass die Armauflage (25) relativ zur Stützeinrichtung (32) bewegbar ist.

8. Orthese nach einem der vorangehenden Ansprüche,
**dadurch gekennzeichnet,**
**dass** von der Verbindungseinrichtung (38) und der Stützeinrichtung (32) eine Linearführung (50) ausgebildet wird, wobei die Linearführung so angeordnet ist, dass eine Abduktion bzw. Adduktion der Armauflage (25) relativ zu der Stützeinrichtung bzw. zu einer Sagittalebene des Rumpfs möglich ist, insbesondere dass an die Stützeinrichtung (32) ein Führungsabschnitt adaptierbar ist, mittels dem die Linearführung (50) verlängerbar ist.

9. Orthese nach einem der vorangehenden Ansprüche,
**dadurch gekennzeichnet,**
**dass** die Verbindungseinrichtung (38) eine Rotationsführung (58) ausbildet, derart, dass eine Rotation der Armauflage (25) in einer Transversalebene des Rumpfs möglich ist, insbesondere dass die Rotationsführung (58) eine Rotation in einem Bereich von +30 Grad bis -30 Grad relativ zu der Stützeinrichtung (32) bzw. zu einer Sagittalebene des Rumpfs ermöglicht.

10. Orthese nach einem der vorangehenden Ansprüche,
**dadurch gekennzeichnet,**
**dass** der Schultergurt (23) und der Brustgurt (22) jeweils mit der Armauflage (25) lösbar verbindbar ist.

11. Orthese nach einem der vorangehenden Ansprüche,
**dadurch gekennzeichnet,**
**dass** die Verbindungseinrichtung (38) eine Kippführung ausbildet, derart, dass ein Kippen der Armauflage (25) relativ zu der Stützeinrichtung (32) bzw. zu einer Transversalebene des Rumpfs möglich ist, insbesondere dass das Kippen der Armauflage (25) mittels einer Verstellung des Schultergurts (23) oder des Brustgurts (22) möglich ist.

12. Verfahren zum Anlegen einer Orthese (10) nach einem der vorangehenden Ansprüche, wobei der Arm an der Positioniervorrichtung fixiert wird, wobei die Bauchgurteinrichtung am Rumpf, diesen in einer Transversalebene umgebend, fixiert wird, wobei ein Schultergurt (23) der Schultergurteinrichtung, der mit einem proximalen Ende (29) der Positioniervorrichtung verbunden ist, über eine dem Arm abgewandte Schulter der Person gezogen wird, und wobei ein mit dem Schultergurt verbundener Brustgurt (22) der Schultergurteinrichtung, der an einem ventralen Abschnitt (26) der Bauchgurteinrichtung angelenkt ist, mit einem distalen Ende (24) der Positioniervorrichtung verbunden wird.

13. Verfahren nach Anspruch 12, wobei der Arm durch eine Verstellung des Brustgurtes in seiner Länge ausbalanciert wird.

## Claims

1. An orthosis (10) for supporting an arm of a person, comprising a securing device (11) for securing the orthosis to a torso of the person, and a positioning device (12) for supporting and fixing the arm, in particular the forearm, elbow and/or upper arm, relative to a person's shoulder associated to that arm, the positioning device being supported by the securing device, the securing device comprising an abdominal strap device (13) and a shoulder strap device (14), the abdominal strap device surrounding the torso in a transversal plane when the orthosis is placed on a person, and the shoulder strap device running over a person's shoulder opposite of that arm, wherein the shoulder strap device comprises a shoulder strap (23) and a chest strap (22) connected to said shoulder strap, said straps being hinged to a distal end (24) and to a proximal end (29) of the positioning device and to a ventral portion (26) of the abdominal strap device, wherein the shoulder strap (23) runs between the proximal end (29) of the positioning device (12) and the chest strap (22)
**characterized in that**
the chest strap (22) runs between the distal end (24) of the positioning device (12) and the ventral portion (26) of the abdominal strap device (13), and wherein the positioning device (12) comprises a support device (32) that is attached to a lateral portion (33) of the abdominal strap device (13), and the positioning device comprises an arm rest (25), an arm of a person being fixable to the arm rest, and the support device being connected to the arm rest by means of a connecting device (38).

2. The orthosis according to any of the preceding claims,
**characterized in that**
the shoulder strap (23) can be arranged on the connected chest strap (22) so as to be displaceable along the chest strap.

3. The orthosis according to any of the preceding claims,
**characterized in that**
the chest strap (22) is length-adjustable.

4. The orthosis according to any of the preceding claims,
**characterized in that**
the chest strap (22) can be arranged on an abdominal strap (15) of the connected abdominal strap device (13) so as to be displaceable along the abdominal strap in the ventral portion (26).

5. The orthosis according to any of the preceding claims,
**characterized in that**
the connecting device (38) is formed such that the arm rest (25) can be fixed in a releasable manner relative to the support device (32).

6. The orthosis according to any of the preceding claims,
**characterized in that**
at the arm rest (25), a hand rest (42) is arranged, which is displaceable relative to the arm rest in a longitudinal direction of the arm rest.

7. The orthosis according to any of the preceding claims,
**characterized in that**
the connecting device (38) is formed such that the arm rest (25) is movable relative to the support device (32).

8. The orthosis according to any of the preceding claims,
**characterized in that**
a linear guide (50) is formed by the connecting device (38) and the support device (32), said linear guide being arranged such that an abduction or adduction of the arm rest (25) is possible relative to the support device and/or to a sagittal plane of the torso, in particular that a guiding portion is adaptable to the support device (32), by means of which guiding portion the linear guide (50) can be extended.

9. The orthosis according to any of the preceding claims,
**characterized in that**
the connecting device (38) forms a rotation guide (58) in such a manner that a rotation of the arm rest (25) is possible in a transversal plane of the torso, in particular that the rotation guide (58) allows a rotation in a range from +30 degrees to -30 degrees relative to the support device (32) and/or to a sagittal plane of the torso.

10. The orthosis according to any of the preceding claims,
**characterized in that**
the shoulder strap (23) and the chest strap (22) can each be detachably connected to the arm rest (25).

11. The orthosis according to any of the preceding claims,
**characterized in that**
the connecting device (38) forms a tilting guide in such a manner that a tilting of the arm rest (25) is possible relative to the support device (32) and/or to a transversal plane of the torso, in particular that the tilting of the arm rest (25) is possible by adjusting the shoulder strap (23) or the chest strap (22).

12. A method for the placement of an orthosis (10) according to any of
the preceding claims,
wherein the arm is fixed to the positioning device, the abdominal strap device being fixed to the torso, surrounding the latter in a transversal plane, wherein a shoulder strap (23) of the shoulder strap device, which is connected to a proximal end (29) of the positioning device, is pulled over the person's shoulder opposite of that arm, and wherein a chest strap (22) of the shoulder strap device, which is connected to the shoulder strap and is hinged to a ventral portion (26) of the abdominal strap device, is connected to a distal end (24) of the positioning device.

13. The method according to claim 12,
wherein the arm is balanced by adjusting the length of the chest strap.

## Revendications

1. Orthèse (10) destinée à supporter un bras d'une personne, comprenant un dispositif de fixation (11) destiné à fixer l'orthèse à un tronc de la personne et un dispositif de positionnement (12) destiné à supporter et fixer le bras, notamment le bras inférieur, le coude et/ou le bras supérieur, par rapport à une épaule d'une personne associée à ce bras, le dispositif de positionnement étant supporté par le dispositif de fixation, le dispositif de fixation comprenant un moyen de sangle abdominale (13) et un moyen de sangle d'épaule (14), le moyen de sangle abdominale entourant le tronc dans un plan transversal tandis que l'orthèse est posée sur une personne, et le moyen de sangle d'épaule s'étendant à travers une épaule de la personne en face de ce bras, le moyen de sangle d'épaule comprenant une sangle d'épaule (23) et une sangle de poitrine (22) liée à ladite sangle d'épaule, qui sont articulées à une extrémité distale (24) et à une extrémité proximale (29) du dispositif de positionnement et à une partie ventrale (26) du moyen de sangle abdominale, la sangle d'épaule (23) s'étendant entre l'extrémité proximale (29) du dispositif de positionnement (12) et la sangle de poitrine (22),
**caractérisée en ce que**
la sangle de poitrine (22) s'étend entre l'extrémité distale (24) du dispositif de positionnement (12) et la partie ventrale (26) du moyen de sangle abdominale (13), et le dispositif de positionnement (12) comprenant un dispositif de support (32) qui est attaché à une partie latérale (33) du moyen de sangle abdominale (13), et le dispositif de positionnement comprenant un appui-bras (25), un bras d'une personne pouvant être fixé à l'appui-bras, et le dispositif de support étant lié à l'appui-bras par l'intermédiaire d'un dispositif de liaison (38).

2. Orthèse selon l'une quelconque des revendications précédentes,
**caractérisée en ce que**
la sangle d'épaule (23) peut être disposée, de manière déplaçable le long de la sangle de poitrine, sur la sangle de poitrine (22) liée.

3. Orthèse selon l'une quelconque des revendications précédentes,
**caractérisée en ce que**
la sangle de poitrine (22) est réglable en longueur.

4. Orthèse selon l'une quelconque des revendications précédentes,
**caractérisée en ce que**
la sangle de poitrine (22) peut être disposée sur une sangle abdominale (15) du moyen de sangle abdominale (13) lié, la sangle de poitrine étant déplaçable le long de la sangle abdominale dans la partie ventrale (26).

5. Orthèse selon l'une quelconque des revendications précédentes,
**caractérisée en ce que**
le dispositif de liaison (38) est formé de telle manière que l'appui-bras (25) peut être fixé de manière amovible par rapport au dispositif de support (32).

6. Orthèse selon l'une quelconque des revendications précédentes,
**caractérisée en ce qu'**
un appui-main (42) est disposé sur l'appui-bras (25), l'appui-main étant déplaçable par rapport à l'appui-bras dans une direction longitudinale de l'appui-bras.

7. Orthèse selon l'une quelconque des revendications précédentes,
**caractérisée en ce que**
le dispositif de liaison (38) est formé de telle manière que l'appui-bras (25) est mobile par rapport au dispositif de support (32).

8. Orthèse selon l'une quelconque des revendications précédentes,
**caractérisée en ce qu'**
un guidage linéaire (50) est formé par le dispositif de liaison (38) et par le dispositif de support (32), ledit guidage linéaire étant disposé de telle manière qu'une abduction et une adduction de l'appui-bras (25) sont possibles par rapport au dispositif de support et à un plan sagittal du tronc, notamment qu'une partie de guidage est adaptable au dispositif de support (32), à l'aide de laquelle le guidage linéaire (50) peut être prolongé.

9. Orthèse selon l'une quelconque des revendications précédentes,
**caractérisée en ce que**
le dispositif de liaison (38) forme un guidage de rotation (58) de telle manière qu'une rotation de l'appui-bras (25) est possible dans un plan transversal du tronc, notamment que le guidage de rotation (58) permet une rotation dans une plage de +30 degrés à -30 degrés par rapport au dispositif de support (32) et à un plan sagittal du tronc.

10. Orthèse selon l'une quelconque des revendications précédentes,
**caractérisée en ce que**
la sangle d'épaule (23) et la sangle de poitrine (22) peuvent chacune être liées de manière amovible à l'appui-bras (25).

11. Orthèse selon l'une quelconque des revendications précédentes,
**caractérisée en ce que**
le dispositif de liaison (38) forme un guidage de basculement de telle manière qu'un basculement de l'appui-bras (25) est possible par rapport au dispositif de support (32) et à un plan transversal du tronc, notamment que le basculement de l'appui-bras (25) est possible par un ajustement de la sangle d'épaule (23) ou de la sangle de poitrine (22).

12. Procédé de pose d'une orthèse (10) selon l'une quelconque des
revendications précédentes,
dans lequel le bras est fixé au dispositif de positionnement, le moyen de sangle abdominale étant fixé au tronc, le moyen de sangle abdominale entourant ce dernier dans un plan transversal, une sangle d'épaule (23) du moyen de sangle d'épaule, qui est liée à une extrémité proximale (29) du dispositif de positionnement, étant tirée à travers une épaule de la personne en face de ce bras, et une sangle de poitrine (22) du moyen de sangle d'épaule, qui est liée à la sangle d'épaule et articulée à une partie ventrale (26) du moyen de sangle abdominale, étant liée à une extrémité distale (24) du dispositif de positionnement.

13. Procédé selon la revendication 12,
dans lequel le bras est équilibré en réglant la longueur de la sangle de poitrine.
